# EUROPÄISCHE PATENTSCHRIFT

(11) **EP 3 272 731 B1**
(45) Veröffentlichungstag und Bekanntmachung des Hinweises auf die Patenterteilung: **01.05.2019**
(21) Anmeldenummer: 16180048.7
(22) Anmeldetag: 19.07.2016
(51) Int. Cl.: C07C 67/38, C07C 69/34, C07C 69/612, B01J 27/13

(54) **VERFAHREN ZUR HERSTELLUNG VON DI- ODER TRICARBONSÄUREESTERN DURCH ALKOXYCARBONYLIERUNG VON DIENEN MIT KONJUGIERTEN DOPPELBINDUNGEN**
METHOD FOR THE PREPARATION OF DI- OR TRICARBOXYLIC ACID ESTERS BY ALKOXYCARBONYLATION OF DIENES USING CONJUGATED DOUBLE BONDS
PROCEDE DE PRODUCTION D'ESTERS D'ACIDES DI- OU TRICARBOXYLIQUES PAR ALKOXY-CARBONYLATION EN UTILISANT DES LIAISONS DOUBLES CONJUGUEES

(43) Veröffentlichungstag der Anmeldung: 24.01.2018
(73) Patentinhaber: Evonik Degussa GmbH, 45128 Essen (DE)
(72) Erfinder: DONG, Kaiwu, 236800 Bo Zhou (CN); JACKSTELL, Ralf, 27478 Cuxhaven Altenwalde (DE); BELLER, Matthias, 18211 Ostseebad Nienhagen (DE); FRIDAG, Dirk, 45721 Haltern am See (DE); HESS, Dieter, 45770 Marl (DE); DYBALLA, Katrin Marie, 45657 Recklinghausen (DE)

(56) Entgegenhaltungen:
- US-A1- 2006 235 241
- US-A1- 2009 131 630
- XIANJIE FANG ET AL: "Palladium-catalysed alkoxycarbonylation of conjugated dienes under acid-free conditions: atom-economic synthesis of [beta],[gamma]-unsaturated esters", ANGEWANDTE CHEMIE, INTERNATIONAL EDITION, Bd. 53, Nr. 34, 1. Juli 2014 (2014-07-01), Seiten 9030-9034, XP055330056, Wiley-VCH Verlag, Weinhem, DE ISSN: 1433-7851, DOI: 10.1002/anie.201404563

## Beschreibung

Die vorliegende Erfindung betrifft ein neues Verfahren zur Herstellung von Di- oder Tricarbonsäureestern durch Alkoxycarbonylierung von Dienen mit konjugierten Doppelbindungen.

Diene mit konjugierten Doppelbindungen (1,3-Diene), insbesondere 1,3-Butadien und Isopren, finden vielfältige Anwendung in der chemischen Industrie und dienen beispielsweise als Grundstoff zur Herstellung von Synthesekautschuk. Eine weitere vielversprechende Anwendung ist die Alkoxycarbonylierung von 1,3-Dienen zu Dicarbonsäuren und den entsprechenden Estern.

Unter einer Alkoxycarbonylierung versteht man die Umsetzung von ethylenisch ungesättigten Verbindungen, wie Dienen, mit Kohlenmonoxid und Alkoholen in Gegenwart eines Metalls bzw. eines Metallkomplexes und eines Liganden zu den entsprechenden Estern. Das folgende Schema zeigt die allgemeine Reaktionsgleichung der Alkoxycarbonylierung einer ethylenisch ungesättigten Verbindung.

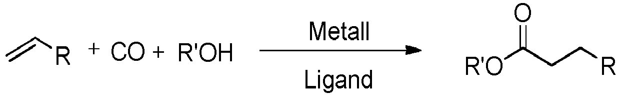

Es ist bekannt, dass unter Anwendung dieses Verfahrens beispielsweise 1,3-Butadien zu Dimethyl-Adipinsäureester, einem wichtigen Grundstoff zur Nylonsynthese, umgesetzt werden kann. Die Synthese von Dimethyl-Adipinsäureester vollzieht sich dabei in zwei Schritten, wobei zunächst 1,3-Butadien zu dem β,γ-ungesättigtem Ester Methyl-3-pentenoat umgesetzt wird, welcher anschließend zu Dimethyl-Adipinsäureester alkoxycarbonyliert wird [Fang, X., Li, H., Jackstell, R. and Beller, M. (2014), Palladium-Catalyzed Alkoxycarbonylation of Conjugated Dienes under Acid-Free Conditions: Atom-Economic Synthesis of β,γ-Unsaturated Esters. Angew. Chem. Int. Ed., 53: 9030-9034]. Ein Nachteil dieser Reaktion ist jedoch, dass sie in zwei Schritten erfolgt und in jedem Schritt ein jeweils anderer Katalysatorkomplex eingesetzt werden muss. Das produziert unnötig Abfall und verursacht hohe Kosten bei Herstellprozess.

Vor diesem Hintergrund hat die vorliegende Erfindung die Aufgabe, ein neues Verfahren zur Herstellung von Di- oder Tricarbonsäureestern, wie beispielsweise Dimethyl-Adipinsäureester, bereitzustellen, mit dem sich bereits in einem Reaktionsschritt hohe Ausbeuten des Di- oder Tricarbonsäureesters erzielen lassen.

Diese Aufgabe wird gelöst durch ein Verfahren umfassend die Verfahrensschritte:
a) Vorlegen eines Diens mit zwei konjugierten Doppelbindungen;
b) Zugabe eines Phosphinliganden und einer Katalysatorvorstufe, welche ausgewählt ist aus Palladiumdichlorid (PdCl₂), Palladiumdibromid (PdBr₂), Palladiumdiiodid (Pdl₂), Palladium(II)-acetylacetonat [Pd(acac)₂], Palladium(II)-acetat [Pd(OAc)₂], Bis-(di-benzylidene-aceton)-palladium [Pd(dba)₂], Bis(acetonitrile)-dichloro-palladium(II) [Pd(CH₃CN)₂Cl₂], Palladium-(cinnamyl)-dichlorid [Pd(cinnamyl)Cl₂];
c) Zugabe eines Alkohols;
c') Zugabe einer Säure zum Reaktionsgemisch;
d) Zuführen von CO;
e) Erwärmen des Reaktionsgemisches, wobei das Dien zu einem Di- oder Tricarbonsäureester umgesetzt wird;
wobei der Phosphinligand eine Verbindung gemäß Formel (**I**) ist 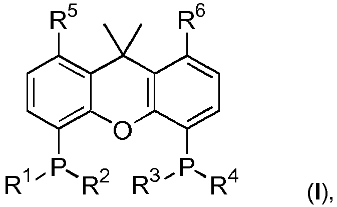 wobei
R¹, R², R³, R⁴ ausgewählt sind aus -(C₁-C₁₂)-Alkyl, -(C₆-C₂₀)-Aryl, -(C₃-C₁₂)-Cycloalkyl, -(C₃-C₁₂)-Heterocycloalkyl, -(C₃-C₂₀)-Heteroaryl;
R⁵, R⁶ ausgewählt sind aus -H, -(C₁-C₁₂)-Alkyl, -(C₆-C₂₀)-Aryl, -(C₃-C₁₂)-Cycloalkyl, -(C₃-C₁₂)-Heterocycloalkyl, -(C₃-C₂₀)-Heteroaryl;
und R¹, R², R³, R⁴, R⁵, R⁶, falls diese für -(C₁-C₁₂)-Alkyl, -(C₆-C₂₀)-Aryl, -(C₃-C₁₂)-Cycloalkyl, - (C₃-C₁₂)-Heterocycloalkyl oder -(C₃-C₂₀)-Heteroaryl stehen, jeweils unabhängig voneinander mit einem oder mehreren Substituenten ausgewählt aus
   -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl, -COO-(C₁-C₁₂)-Alkyl, -COO-(C₃-C₁₂)-Cycloalkyl, -CONH-(C₁-C₁₂)-Alkyl, -CONH-(C₃-C₁₂)-Cycloalkyl, - CO-(C₁-C₁₂)-Alkyl, -CO-(C₃-C₁₂)-Cycloalkyl, -N-[(C₁-C₁₂)-Alkyl]₂, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, -COOH, -OH, -SO₃H, -NH₂, Halogen substituiert sein können.

Hierbei können die Verfahrensschritte a), b), c) und d) in beliebiger Reihenfolge erfolgen. Üblicherweise erfolgt die Zugabe von CO jedoch, nachdem die Reaktionspartner in den Schritten a) bis c) vorgelegt wurden. Die Schritte d) und e) können gleichzeitig oder nacheinander erfolgen. Darüber hinaus kann CO auch in mehreren Schritten zugeführt werden, so dass beispielsweise zunächst ein Teil des CO zugeführt, dann erwärmt und anschließend ein weiterer Teil CO zugeführt wird.

Es hat sich überraschenderweise gezeigt, dass die erfindungsgemäße Kombination von Phosphinliganden und Katalysatorvorstufe die direkte Umsetzung des Diens zu dem entsprechenden Di- oder Tricarbonsäureester ermöglicht, wobei als Nebenprodukt nur geringe Mengen von β,γ-ungesättigtem Monocarbonsäureester erhalten werden. Dadurch können Di- oder Tricarbonsäureester mit dem erfindungsgemäßen Verfahren einfacher hergestellt werden als mit den aus dem Stand der Technik bekannten Alkoxycarbonylierungsverfahren.

Der Begriff (C₁-C₁₂)-Alkyl umfasst geradkettige und verzweigte Alkylgruppen mit 1 bis 12 Kohlenstoffatomen. Vorzugsweise handelt es sich dabei um (C₁-C₈)-Alkylgruppen, besonders bevorzugt (C₁-C₆)-Alkyl, am meisten bevorzugt (C₁-C₄)-Alkyl.

Geeignete (C₁-C₁₂)-Alkylgruppen sind insbesondere Methyl, Ethyl, Propyl, Isopropyl, *n*-Butyl, *iso*-Butyl, *sec*-Butyl, *tert*-Butyl, *n*-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,1-Dimethylbutyl, 1,2-Dimethylbutyl, 2,2-Dimethylbutyl, 1,3-Dimethylbutyl, 2,3-Dimethylbutyl, 3,3-Dimethylbutyl, 1,1,2-Trimethylpropyl, 1,2,2-Trimethylpropyl, 1-Ethylbutyl, 1-Ethyl-2-methylpropyl, n-Heptyl, 2-Heptyl, 3-Heptyl, 2-Ethylpentyl, 1-Propylbutyl, *n*-Octyl, 2-Ethylhexyl, 2-Propylheptyl, Nonyl, Decyl.

Die Erläuterungen zum Begriff (C₁-C₁₂)-Alkyl gelten insbesondere auch für die Alkylgruppen in -O-(C₁-C₁₂)-Alkyl, -S-(C₁-C₁₂)-Alkyl, -COO-(C₁-C₁₂)-Alkyl, -CONH-(C₁-C₁₂)-Alkyl, -CO-(C₁-C₁₂)-Alkyl und -N-[(C₁-C₁₂)-Alkyl]₂.

Der Begriff (C₃-C₁₂)-Cycloalkyl umfasst mono-, bi- oder tricyclische Kohlenwasserstoffgruppen mit 3 bis 12 Kohlenstoffatomen. Vorzugsweise handelt es sich dabei um (C₅-C₁₂)-Cycloalkyl.

Die (C₃-C₁₂)-Cycloalkylgruppen weisen vorzugsweise 3 bis 8, besonders bevorzugt 5 oder 6, Ringatome auf.

Geeignete (C₃-C₁₂)-Cycloalkylgruppen sind insbesondere Cyclopropyl, Cyclobutyl, Cyclopentyl, Cyclohexyl, Cycloheptyl, Cyclooctyl, Cyclododecyl, Cyclopentadecyl, Norbonyl, Adamantyl.

Die Erläuterungen zum Begriff (C₃-C₁₂)-Cycloalkyl gelten insbesondere auch für die Cycloalkylgruppen in -O-(C₃-C₁₂)-Cycloalkyl, -S-(C₃-C₁₂)-Cycloalkyl, -COO-(C₃-C₁₂)-Cycloalkyl, -CONH-(C₃-C₁₂)-Cycloalkyl, -CO-(C₃-C₁₂)-Cycloalkyl.

Der Begriff (C₃-C₁₂)-Heterocycloalkyl umfasst nichtaromatische, gesättigte oder teilweise ungesättigte cycloaliphatische Gruppen mit 3 bis 12 Kohlenstoffatomen, wobei eins oder mehrere der Ringkohlenstoffatome durch Heteroatome ersetzt sind. Die (C₃-C₁₂)-Heterocycloalkylgruppen weisen vorzugsweise 3 bis 8, besonders bevorzugt 5 oder 6, Ringatome auf und sind ggf. mit aliphatischen Seitenketten substituiert. In den Heterocycloalkylgruppen sind im Unterschied zu den Cycloalkylgruppen eins oder mehrere der Ringkohlenstoffatome durch Heteroatome oder heteroatomhaltige Gruppen ersetzt. Die Heteroatome oder die heteroatomhaltigen Gruppen sind vorzugsweise ausgewählt unter O, S, N, N(=O), C(=O), S(=O). Eine (C₃-C₁₂)-Heterocycloalkylgruppe im Sinne dieser Erfindung ist somit auch Ethylenoxid.

Geeignete (C₃-C₁₂)-Heterocycloalkylgruppen sind insbesondere Tetrahydrothiophenyl, Tetrahydrofuryl, Tetrahydropyranyl und Dioxanyl.

Der Begriff (C₆-C₂₀)-Aryl umfasst mono- oder polycyclische aromatische Kohlenwasserstoffreste mit 6 bis 20 Kohlenstoffatomen. Vorzugsweise handelt es sich dabei um (C₆-C₁₄)-Aryl, besonders bevorzugt (C₆-C₁₀)-Aryl.

Geeignete (C₆-C₂₀)-Arylgruppen sind insbesondere Phenyl, Naphthyl, Indenyl, Fluorenyl, Anthracenyl, Phenanthrenyl, Naphthacenyl, Chrysenyl, Pyrenyl, Coronenyl. Bevorzugte (C₆-C₂₀)-Arylgruppen sind Phenyl, Naphthyl und Antracenyl.

Der Begriff (C₃-C₂₀)-Heteroaryl umfasst mono- oder polycyclische aromatische Kohlenwasserstoffreste mit 3 bis 20 Kohlenstoffatomen, wobei eins oder mehrere der Kohlenstoffatome durch Heteroatome ersetzt sind. Bevorzugte Heteroatome sind N, O und S. Die (C₃-C₂₀)-Heteroarylgruppen weisen 3 bis 20, bevorzugt 6 bis 14, besonders bevorzugt 6 bis 10 Ringatome auf.

Geeignete (C₃-C₂₀)-Heteroarylgruppen sind insbesondere Furyl, Thienyl, Pyrrolyl, Oxazolyl, Isoxazolyl, Thiazolyl, Isothiazolyl, Imidazolyl, Pyrazolyl, Furazanyl, Tetrazolyl, Pyridyl, Pyridazinyl, Pyrimidyl, Pyrazinyl, Benzofuranyl, Indolyl, Isoindolyl, Benzimidazolyl, Chinolyl, Isochinolyl.

Der Begriff Halogen umfasst insbesondere Fluor, Chlor, Brom und lod. Besonders bevorzugt sind Fluor und Chlor.

In einer Ausführungsform können die Reste R¹, R², R³, R⁴, R⁵, R⁶, falls diese für -(C₁-C₁₂)-Alkyl, -(C₆-C₂₀)-Aryl, -(C₃-C₁₂)-Cycloalkyl, -(C₃-C₁₂)-Heterocycloalkyl oder -(C₃-C₂₀)-Heteroaryl stehen, jeweils unabhängig voneinander mit einem oder mehreren Substituenten ausgewählt aus -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl, - (C₃-C₂₀)-Heteroaryl-(C₁-C₁₂)-Alkyl, -(C₃-C₂₀)-Heteroaryl-O-(C₁-C₁₂)-Alkyl, -COOH, -OH, -SO₃H, -NH₂, Halogen substituiert sein.

In einer Ausführungsform können die Reste R¹, R², R³, R⁴, R⁵, R⁶, falls diese für -(C₁-C₁₂)-Alkyl, -(C₆-C₂₀)-Aryl, -(C₃-C₁₂)-Cycloalkyl, -(C₃-C₁₂)-Heterocycloalkyl oder -(C₃-C₂₀)-Heteroaryl stehen, jeweils unabhängig voneinander mit einem oder mehreren Substituenten ausgewählt aus -(C₁-C₁₂)-Alkyl, -(C₃-C₁₂)-Cycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl substituiert sein.

In einer Ausführungsform können die Reste R¹, R², R³, R⁴, R⁵, R⁶, falls diese für -(C₁-C₁₂)-Alkyl, -(C₆-C₂₀)-Aryl, -(C₃-C₁₂)-Cycloalkyl, -(C₃-C₁₂)-Heterocycloalkyl oder -(C₃-C₂₀)-Heteroaryl stehen, jeweils unabhängig voneinander mit einem oder mehreren Substituenten ausgewählt aus -(C₁-C₁₂)-Alkyl, -(C₃-C₁₂)-Cycloalkyl, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl substituiert sein.

In einer Ausführungsform können die Reste R¹, R², R³, R⁴, R⁵, R⁶, falls diese für -(C₁-C₁₂)-Alkyl, -(C₆-C₂₀)-Aryl, -(C₃-C₁₂)-Cycloalkyl, -(C₃-C₁₂)-Heterocycloalkyl oder -(C₃-C₂₀)-Heteroaryl stehen, jeweils unabhängig voneinander mit einem oder mehreren Substituenten ausgewählt aus -(C₁-C₁₂)-Alkyl, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl substituiert sein.

In einer Ausführungsform sind die Reste R¹, R², R³, R⁴, R⁵, R⁶, falls diese für -(C₁-C₁₂)-Alkyl, - (C₆-C₂₀)-Aryl, -(C₃-C₁₂)-Cycloalkyl, -(C₃-C₁₂)-Heterocycloalkyl oder -(C₃-C₂₀)-Heteroaryl stehen, unsubstituiert.

In einer bevorzugten Ausführungsform sind R¹, R², R³, R⁴ ausgewählt aus -(C₁-C₁₂)-Alkyl, -(C₆-C₂₀)-Aryl, -(C₃-C₁₂)-Cycloalkyl, -(C₃-C₂₀)-Heteroaryl; besonders bevorzugt aus -(C₁-C₁₂)-Alkyl, -(C₆-C₂₀)-Aryl, -(C₃-C₂₀)-Heteroaryl. Am meisten bevorzugt sind R¹, R², R³, R⁴ jeweils -(C₆-C₂₀)-Aryl. Dabei können R¹, R², R³, R⁴ wie oben beschrieben substituiert sein. Vorzugsweise sind R¹, R², R³, R⁴ dabei jedoch unsubstituiert.

In einer bevorzugten Ausführungsform sind R⁵, R⁶ ausgewählt aus -H, -(C₁-C₁₂)-Alkyl, -(C₆-C₂₀)-Aryl; besonders bevorzugt aus -H, -(C₁-C₁₂)-Alkyl. Am meisten bevorzugt sind R⁵, R⁶ jeweils -H. Dabei können R⁵, R⁶, falls diese -(C₁-C₁₂)-Alkyl oder -(C₆-C₂₀)-Aryl darstellen wie oben beschrieben substituiert sein. Vorzugsweise sind R⁵, R⁶ jedoch unsubstituiert, falls sie - (C₁-C₁₂)-Alkyl oder -(C₆-C₂₀)-Aryl darstellen.

In einer bevorzugten Ausführungsform ist der Phosphinligand eine Verbindung gemäß Formel (**1**):

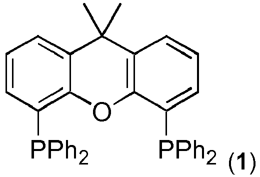

Das in dem erfindungsgemäßen Verfahren als Edukt eingesetzte Dien umfasst wenigstens zwei konjugierte Doppelbindungen. Das Dien kann mit einer oder mehreren Alkyl-, Alkenyl, Alkinyl- oder Arylgruppen substituiert sein. Vorzugsweise umfasst das Dien insgesamt 4 bis 30 Kohlenstoffatome, bevorzugt 4 bis 22 Kohlenstoffatome, am meisten bevorzugt 4 bis 12 Kohlenstoffatome.

Das Dien kann auch mehr als zwei Doppelbindungen umfassen, wobei zwei davon konjugiert sein müssen. Im Fall, dass das Dien drei oder mehr Doppelbindungen umfasst, hat sich gezeigt, dass es in der erfindungsgemäßen Reaktion auch direkt zu einem Tricarbonsäureester umgesetzt werden kann. Somit umfasst die erfindungsgemäße Reaktion auch die Umsetzung des Diens zu einem Tricarbonsäureester.

Das Dien kann zusätzliche funktionelle Gruppen enthalten. Vorzugsweise umfasst das Dien eine oder mehrere funktionelle Gruppen ausgewählt aus Carboxyl-, Thiocarboxyl-, Sulfo-, Sulfinyl-, Carbonsäureanhydrid-, Imid-, Carbonsäureester-, Sulfonsäureester-, Carbamoyl-, Sulfamoyl-, Cyano-, Carbonyl-, Carbonothioyl-, Hydroxyl-, Sulfhydryl- , Amino-, Ether-, Thioether-, Aryl-, Heteroaryl- oder Silylgruppen und/oder Halogensubstituenten. In einer Ausführungsform umfasst das Dien keine dieser funktionellen Gruppen.

Geeignete Diene sind insbesondere 1,3-Butadien, Isopren, 1,3-Pentadien, 2,3-Dimethyl-1,3-butadien, 2,4-Dimethyl-1,3-pentadien, 7-Methyl-3-methylen-1,6-octadien (Myrcen) oder 2-Phenyl-1,3-butadien.

In einer Ausführungsform ist das Dien ausgewählt aus Verbindungen der Formel (**II**) 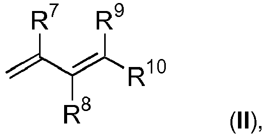 wobei R⁷, R⁸, R⁹, R¹⁰ unabhängig voneinander ausgewählt sein können aus -H, -(C₁-C₁₂)-Alkyl und -(C₆-C₂₀)-Aryl. Besonders bevorzugt sind R⁷, R⁸, R⁹, R¹⁰ unabhängig voneinander ausgewählt aus -H, -(C₁-C₆)-Alkyl und Phenyl. Am meisten bevorzugt sind R⁷, R⁸, R⁹, R¹⁰ unabhängig voneinander ausgewählt aus -H, Methyl, Ethyl und Phenyl. Vorzugsweise sind die Reste R⁸, R⁹ -H. Besonders bevorzugt sind die Reste R⁸, R⁹, R¹⁰ -H.

In einer besonders bevorzugten Ausführungsform ist das Dien ausgewählt aus 1,3-Butadien, Isopren, 2,3-Dimethyl-1,3-butadien, 2,4-Dimethyl-1,3-pentadien oder 2-Phenyl-1,3-butadien. Am meisten bevorzugt ist das Dien Isopren.

Die erfindungsgemäße Alkoxycarbonylierung wird durch einen Pd-Komplex katalysiert. Der Pd-Komplex wird dabei *in situ* aus einer Verbindung, welche Pd umfasst, und dem freien Phosphinliganden gebildet. Dabei wird die Verbindung, welche Pd umfasst, auch als Katalysatorvorstufe bezeichnet.

Die Katalysatorvorstufe ist ausgewählt aus Palladiumdichlorid (PdCl₂), Palladiumdibromid (PdBr₂), Palladiumdiiodid (Pdl₂), Palladium(II)-acetylacetonat [Pd(acac)₂], Palladium(II)-acetat [Pd(OAc)₂], Bis-(di-benzylidene-aceton)-palladium [Pd(dba)₂], Bis(acetonitrile)-dichloro-palladium(II) [Pd(CH₃CN)₂Cl₂], Palladium-(cinnamyl)-dichlorid [Pd(cinnamyl)Cl₂],

Vorzugsweise ist die Katalysatorvorstufe ausgewählt aus Palladiumdichlorid (PdCl₂), Palladiumdibromid (PdBr₂), Palladiumdiiodid (PdI₂, Palladium(II)-acetylacetonat [Pd(acac)₂], Palladium(II)-acetat [Pd(OAc)₂], Bis-(di-benzylidene-aceton)-palladium [Pd(dba)₂].

Besonders bevorzugt ist die Katalysatorvorstufe ausgewählt aus Palladiumdichlorid (PdCl₂), Palladiumdibromid (PdBr₂) und Palladiumdiiodid (PdI₂). Am meisten bevorzugt ist Palladiumdibromid (PbBr₂).

Der Alkohol in Verfahrensschritt c) umfasst vorzugsweise 1 bis 30 Kohlenstoffatome, bevorzugt 1 bis 22 Kohlenstoffatome, besonders bevorzugt 1 bis 12 Kohlenstoffatome. Der Alkohol kann beispielsweise linear, verzweigt, alizyklisch oder zyklisch sein. Der Alkohol kann auch ungesättigte oder aromatische Gruppen umfassen. Der Alkohol kann eine oder mehrere Hydroxylgruppen umfassen.

Der Alkohol kann zusätzlich zu der einen oder mehreren Hydroxylgruppen weitere funktionelle Gruppen enthalten. Vorzugsweise kann der Alkohol zusätzlich eine oder mehrere funktionelle Gruppen ausgewählt aus Carboxyl-, Thiocarboxyl-, Sulfo-, Sulfinyl-, Carbonsäureanhydrid-, Imid-, Carbonsäureester-, Sulfonsäureester-, Carbamoyl-, Sulfamoyl-, Cyano-, Carbonyl-, Carbonothioyl-, Sulfhydryl-, Amino-, Ether-, Thioether-, oder Silylgruppen, und/oder Halogensubstituenten enthalten.

In einer Ausführungsform umfasst der Alkohol keine weiteren funktionellen Gruppen außer Hydroxylgruppen.

In einer Ausführungsform umfasst der Alkohol nur eine Hydroxylgruppe.

Geeignete Alkohole sind beispielsweise Alkanole oder Cycloalkanole, insbesondere Methanol, Ethanol, 1-Propanol, iso-Propanol, n-Butanol, iso-Butanol, sec-Butanol, tert-Butanol, 1-Pentanol, 2-Pentanol, 3-Pentanol, 1-Hexanol, Cyclohexanol, 2-Ethylhexanol, iso-Nonanol, 2-Propylheptanol.

Geeignete Alkohole sind auch Aryl-substituierte Alkanole, wie z.B. Benzylalkohol, 1-Phenylethanol oder 2-Phenylethanol.

Geeignete Alkohole mit mehr als einer Hydroxylgruppe sind beispielsweise Cyclohexan-1,2-diol, 1,2-Ethandiol, 1,3-Propandiol, Glycerol, 1,2,4-Butantriol, 2-Hydroxymethyl-1,3-Propandiol, 1,2,6-Trihydroxyhexan, Pentaerythritol, 1,1,1-Tri(hydroxymethyl)ethan, Brenzkatechin, Resorcin und Hydroxyhydrochinon.

Geeignete Alkohole sind auch Sucrose, Fructose, Mannose, Sorbose, Galactose und Glucose.

In einer Ausführungsform ist der Alkohol in Verfahrensschritt c) ausgewählt aus Methanol, Ethanol, 1-Propanol, iso-Propanol, n-Butanol, iso-Butanol, sec-Butanol, tert-Butanol, 1-Pentanol, 2-Pentanol, 3-Pentanol, 1-Hexanol, Cyclohexanol, 2-Ethylhexanol, iso-Nonanol, 2-Propylheptanol, Benzylalkohol, 1-Phenylethanol oder 2-Phenylethanol.

Vorzugsweise ist der Alkohol in Verfahrensschritt c) ausgewählt aus Methanol, Ethanol, 1-Propanol, iso-Propanol, n-Butanol, iso-Butanol, sec-Butanol, tert-Butanol oder 2-Phenylethanol.

Besonders bevorzugt ist der Alkohol in Verfahrensschritt c) ausgewählt aus Methanol, Ethanol, iso-Propanol, n-Butanol oder 2-Phenylethanol.

Am meisten bevorzugt ist der Alkohol in Verfahrensschritt c) Methanol.

In einer Variante des Verfahrens wird der Alkohol im Verfahrensschritt c) im Überschuss eingesetzt.

In einer Variante des Verfahrens wird der Alkohol im Verfahrensschritt c) gleichzeitig als Lösungsmittel eingesetzt.

In einer Variante des Verfahrens wird ein weiteres Lösungsmittel verwendet, welches beispielsweise ausgewählt ist aus: Toluol, Xylol, Hexan, Heptan, Tetrahydrofuran (THF) oder Methylenchlorid (CH₂Cl₂). Besonders bevorzugte Lösungsmittel sind Toluol und Xylol (insbesondere p-Xylol).

CO wird in Schritt d) vorzugsweise bei einem CO-Partialdruck zwischen 0,1 und 10 MPa (1 bis 100 bar), bevorzugt zwischen 1 und 8 MPa (10 bis 80 bar), besonders bevorzugt zwischen 3 und 6 MPa (30 bis 60 bar), am meisten bevorzugt zwischen 3,5 und 4,5 MPa (35 bis 45 bar) zugeführt.

Die Reaktionsmischung wird Schritt e) des erfindungsgemäßen Verfahrens vorzugsweise auf eine Temperatur zwischen 10 °C und 180 °C, bevorzugt zwischen 20 und 160 °C, besonders bevorzugt zwischen 80 und 160 °C, am meisten bevorzugt zwischen 130 und 150°C erwärmt, um das Dien zu einem Di- oder Tricarbonsäureester umzusetzen.

Das molare Verhältnis von dem in Schritt a) vorgelegten Dien zu dem in Schritt c) zugegebenen Alkohol beträgt vorzugsweise zwischen 1:1 bis 1:20, bevorzugt 1:2 bis 1:10, besonders bevorzugt 1:3 bis 1:4.

Das Massenverhältnis von Pd zu dem in Schritt a) vorgelegten Dien beträgt vorzugsweise zwischen 0,001 und 0,5 Gew.-%, bevorzugt zwischen 0,01 und 0,1 Gew.-%, besonders bevorzugt zwischen 0,01 und 0,05 Gew.-%.

Die Stoffmenge an Pd bezogen auf die Stoffmenge des in Schritt a) vorgelegten Diens beträgt vorzugsweise zwischen 0,1 und 10 mol-%, bevorzugt 0,5 bis 5 mol-%, besonders bevorzugt 0,8 bis 2 mol-%.

Das molare Verhältnis des Phosphinliganden zu Pd beträgt vorzugsweise zwischen 0,1:1 und 400:1, bevorzugt zwischen 0,5:1 und 400:1, besonders bevorzugt zwischen 1:1 und 100:1, am meisten bevorzugt zwischen 2:1 und 50:1.

Das Verfahren wird unter Zusatz einer Säure durchgeführt. Dabei kann es sich vorzugsweise um eine Brønsted- oder eine Lewis-Säure handeln.

Geeignete Brønsted-Säuren haben vorzugsweise eine Säurestärke von pKₛ ≤ 5, bevorzugt eine Säurestärke von pKₛ ≤ 3. Die angegebene Säurestärke pKₛ bezieht sich auf den bei Normalbedingungen (25°C, 1,01325 bar) bestimmten pKₛ-Wert. Bei einer mehrprotonigen Säure bezieht sich die Säurestärke pKₛ im Rahmen dieser Erfindung auf den pKₛ-Wert des ersten Protolyseschrittes.

Vorzugsweise ist die Säure keine Carbonsäure.

Geeignete Brønsted-Säuren sind beispielsweise Perchlorsäure, Schwefelsäure, Phosphorsäure, Methylphosphonsäure und Sulfonsäuren. Vorzugsweise handelt es sich bei der Säure um Schwefelsäure oder eine Sulfonsäure. Geeignete Sulfonsäuren sind beispielsweise Methansulfonsäure, Trifluormethansulfonsäure, tert-Butansulfonsäure, p-Toluolsulfonsäure (PTSA), 2-Hydroxypropan-2-sulfonsäure, 2,4,6-Trimethylbenzolsulfonsäure und Dodecylsulfonsäure. Besonders bevorzugte Säuren sind Schwefelsäure, Methansulfonsäure, Trifluormethansulfonsäure und p-Toluolsulfonsäure.

Als Lewis-Säure kann beispielsweise Aluminiumtriflat eingesetzt werden.

In einer Ausführungsform beträgt die Menge an in Schritt c') zugesetzter Säure 0,3 bis 40 mol- %, bevorzugt 0,4 bis 15 mol-%, besonders bevorzugt 0,5 bis 5 mol-%, am meisten bevorzugt 0,6 bis 3 mol-%, bezogen auf die Stoffmenge des in Schritt a) vorgelegten Diens.

### Beispiele

Die folgenden Beispiele veranschaulichen die Erfindung.

### Allgemeine Arbeitsvorschriften

Soweit nicht anders angegeben, beziehen sich relative Stoffmengen in mol-% auf die Stoffmenge an Olefin (Substrat).

Alle Reaktionsprodukte wurden durch Säulenchromatographie über Silica Gel 60, 0,063-0,2 mm, 70-230 mesh der Firma Merck aus der Reaktionsmischung isoliert.

Gelchromatographische Analysen (GC Analysen) wurden mit einem Instrument des Typs Agilent GC 7890A der Firma Agilent mit einer HP5 Säule (Polydimethylsiloxan mit 5% Phenylgruppen, 30 m, 0.32 mm i.d., 0.25 µm Filmdicke) durchgeführt. Temperaturprogramm: 35°C, 10 min.; 10°C/min bis 285 °C, 5 min. Injektionsvolumen 1 µl mit 50:1 Split. Die Retentionszeiten der gereinigten Produkte sind in der folgenden Tabelle angegeben:

| Produkt | Retentionszeit (min) |
|---|---|
| 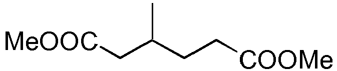 | 22,0 |
| 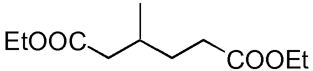 | 23,6 |
| 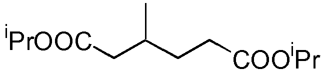 | 24,3 |
| 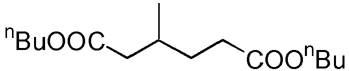 | 27,4 |
| 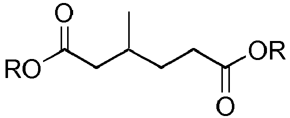 | 35,3 |
| R = CH₂CH₂C₆H₅ | |
| 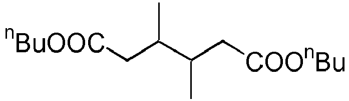 | 28,0 |
| 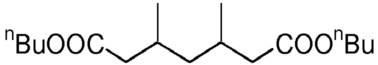 | 28,5 |
| 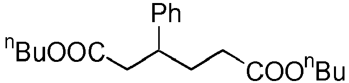 | 31,6 |
| 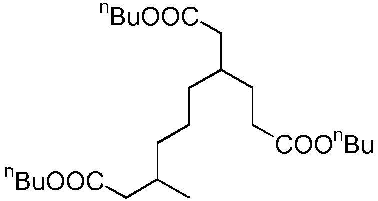 | 35,1 |

### Alkoxycarbonylierung von Isopren mit n-Butanol mit unterschiedlichen Liganden

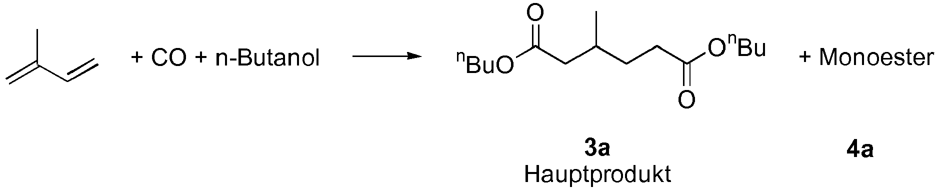

Ein 4 ml Probengläschen wurde mit PdBr₂ (2,64 mg, 1,0 mol-%), PTSA (9,5 mg, 5,0 mol-%), dem jeweiligen Ligand (2.0 mol-%) und einem Magnetrührstab beladen. Das Gefäß wurde mit einem Septum (PTFE-beschichteter Styrol-Butadien-Kautschuk) und einem Phenolharzdeckel verschlossen. Über eine durch das Septum gestochene Kanüle wurde der Gasaustausch zwischen Probengläschen und Umgebung ermöglicht. Das Probengläschen wurde dreimal mit Argon gespült. Para-Xylol (2,0 ml), Isopren (100 µl, 1,0 mmol) und n-Butanol (274 µl, 3,0 mmol) wurden über eine Spritze in das Probengläschen injiziert. Das Probengläschen wurde auf einer Metallplatte platziert und diese unter Argon-Atmosphäre in einen 300 ml Autoklaven des Typs 4560 von Parr Instruments überführt. Nach dreimaligem Spülen des Autoklaven mit CO wurde der CO-Druck bei Raumtemperatur auf 40 bar eingestellt. Die Reaktion lief 48 Stunden bei 120 °C. Nach Abschluss der Reaktion wurde der Autoklav auf Raumtemperatur abgekühlt und vorsichtig entspannt. Isooctan (100 µl) wurde als interner GC-Standard zugesetzt. Die Ausbeute von Dicarbonsäureester (**3a**) und Monocarbonsäureester (**4a**) wurde mittels GC bestimmt. Die Ergebnisse sind in folgender Tabelle zusammengefasst. Bei den eingesetzten Liganden handelt es sich jeweils um kommerziell erhältliche Verbindungen.

| Beispiel | Ligand | Ausbeute **3a** | Ausbeute **4a** |
|---|---|---|---|
| 1 | kein Ligand | - | 68% |
| 2* | 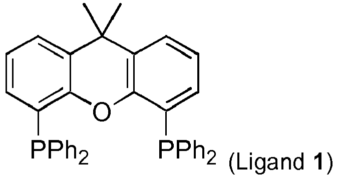 | 89 % | 11% |
| 2 | 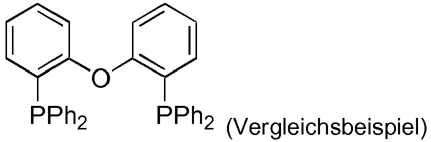 | 0 | 42% |
| 3 | 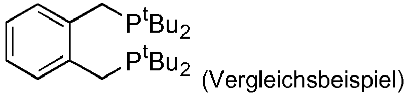 | 0 | 42% |
| 4 | 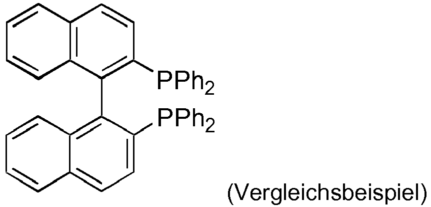 | 0 | 0 |

| | | | |
|---|---|---|---|
| *: erfindungsgemäßes Beispiel | | | |

Wie dieser Versuch zeigt, ist der erfindungsgemäß eingesetzte Ligand **1** als einziger der untersuchten Liganden, in der Lage Isopren in einem Schritt zu einem Dicarbonsäureester umzusetzen. Mit dem im Stand der Technik im Zusammenhang mit der Alkoxycarbonylierung von Isopren beschriebenen Liganden 2 bis 4 (vgl. X. Fang et al, Angew. Chem. Int. Ed., 2014, 53, 9030 - 9034) werden allenfalls Monoester, jedoch keine Dicarbonsäureester erhalten.

### Alkoxycarbonylierung von Isopren mit n-Butanol mit unterschiedlichen Katalysatorvorstufen

Ein 4 ml Probengläschen wurde mit 1.0 mol-% bzw. 0.5 mol-% der jeweiligen Katalysatorvorstufe, PTSA (9,5 mg, 5,0 mol-%), Ligand **1** (11,6 mg, 2.0 mol-%) und einem Magnetrührstab beladen. Das Gefäß wurde mit einem Septum (PTFE-beschichteter Styrol-Butadien-Kautschuk) und einem Phenolharzdeckel verschlossen. Über eine durch das Septum gestochene Kanüle wurde der Gasaustausch zwischen Probengläschen und Umgebung ermöglicht. Das Probengläschen wurde dreimal mit Argon gespült. Para-Xylol (2,0 ml), Isopren (100 µl, 1,0 mmol) und n-Butanol (274 µl, 3,0 mmol) wurden über eine Spritze in das Probengläschen injiziert. Das Probengläschen wurde auf einer Metallplatte platziert und diese unter Argon-Atmosphäre in einen 300 ml Autoklaven des Typs 4560 von Parr Instruments überführt. Nach dreimaligem Spülen des Autoklaven mit CO wurde der CO-Druck bei Raumtemperatur auf 40 bar eingestellt. Die Reaktion lief 48 Stunden bei 140 °C. Nach Abschluss der Reaktion wurde der Autoklav auf Raumtemperatur abgekühlt und vorsichtig entspannt. Isooctan (100 µl) wurde als interner GC-Standard zugesetzt. Die Ausbeute von Dicarbonsäureester (**3a**) und Monoester (**4a**) wurde mittels GC bestimmt. Die Ergebnisse sind in folgender Tabelle zusammengefasst.

| Beispiel | Katalysatorvorstufe | Ausbeute **3a** | Ausbeute **4a** |
|---|---|---|---|
| 1 | 1.0 mol-% PdCl₂ | 71% | 26% |
| 2 | 1.0 mol-% PdBr₂ | 89% | 11% |
| 3 | 1.0 mol-% PdI₂ | 67% | 19% |
| 4 | 1.0 mol-% Pd(OAc)₂ | 41% | 32% |
| 5 | 1.0 mol-% Pd(acac)₂ | 44% | 27% |
| 6 | 0.5 mol-% Pd₂(dba)₃ | 24% | 52% |

Dieser Versuch zeigt, dass mit verschiedenen erfindungsgemäß eingesetzten Katalysatorvorstufen eine direkte Umsetzung von Isopren zu Dicarbonsäureester erreicht werden kann. Die besten Ergebnisse werden dabei mit PdCl₂, PdBr₂, PdI₂ erzielt.

### Alkoxycarbonylierung unterschiedlicher Diene mit unterschiedlichen Alkoholen

Ein 4 ml Probengläschen wurde mit PdBr₂, PTSA, Ligand 1 in den angegebenen Mengen und einem Magnetrührstab beladen. Das Gefäß wurde mit einem Septum (PTFE-beschichteter Styrol-Butadien-Kautschuk) und einem Phenolharzdeckel verschlossen. Über eine durch das Septum gestochene Kanüle wurde der Gasaustausch zwischen Probengläschen und Umgebung ermöglicht. Das Probengläschen wurde dreimal mit Argon gespült. Para-Xylol (2,0 ml), 1,0 mmol Dien und 3,0 mmol Alkohol wurden über eine Spritze in das Probengläschen injiziert. Das Probengläschen wurde auf einer Metallplatte platziert und diese unter Argon-Atmosphäre in einen 300 ml Autoklaven des Typs 4560 von Parr Instruments überführt. Nach dreimaligem Spülen des Autoklaven mit CO wurde der CO-Druck bei Raumtemperatur auf 40 bar eingestellt. Die Reaktion lief 48 bzw. 96 Stunden bei 140 °C. Nach Abschluss der Reaktion wurde der Autoklav auf Raumtemperatur abgekühlt und vorsichtig entspannt. Isooctan (100 µl) wurde als interner GC-Standard zugesetzt. Die Ausbeute des Hauptprodukts wurde mittels GC bestimmt. Die Ergebnisse sind in folgender Tabelle zusammengefasst.

| Beispiel¹⁾ | Dien | Alkohol | Hauptprodukt | Ausbeute |
|---|---|---|---|---|
| 1 | Isopren | Methanol | 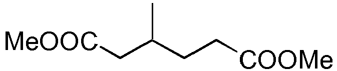 | 76% |
| 2 | Isopren | Ethanol | 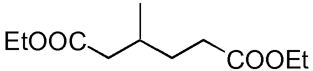 | 82% |
| 3 | Isopren | iso-Propanol | 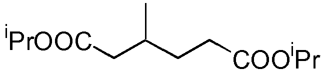 | 70% |
| 4 | Isopren | n-Butanol | 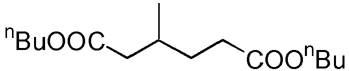 | 94% |
| 5 | Isopren | 2-Phenylethanol | 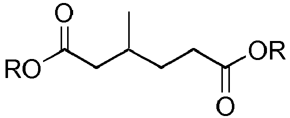 | 94% |
| | | | R = CH₂CH₂C₆H₅ | |
| 6 | 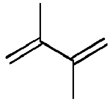 | n-Butanol | 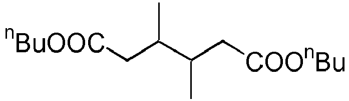 | 79 % |
| 7 | 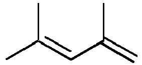 | n-Butanol | 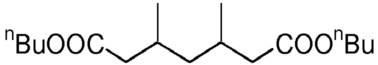 | 99% |
| 8 | 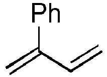 | n-Butanol | 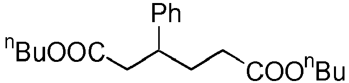 | 30% |
| 9 | 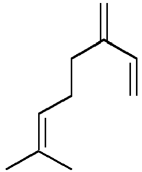 | n-Butanol | 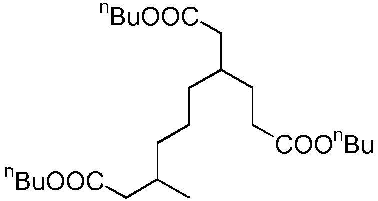 | 37 % |

| | | | | |
|---|---|---|---|---|
| ¹⁾ Beispiele 1 bis 5, 7 und 8: 1,0 mol-% PdBr₂, 2,0 mol-% Ligand **1**, 5,0 mol-% PTSA, 48 h; Beispiele 6 und 9: 2,0 mol-% PdBr₂, 4,0 mol-% Ligand **1**, 10,0 mol-% PTSA, 96 h. | | | | |

Die oben beschriebenen Beispiele zeigen, dass 1,3-Diene, wie beispielsweise Isopren, mit dem erfindungsgemäßen Verfahren direkt zu Di- oder Tricarbonsäureestern umgesetzt werden können. Die ist ein Vorteil gegenüber dem aus dem Stand der Technik zweischrittigen Verfahren zur Herstellung von Dicarbonsäureestern. Auch ein Einsatz verschiedener Alkohole konnte erfolgreich gezeigt werden.

## Patentansprüche

1. Verfahren umfassend die Verfahrensschritte:
a) Vorlegen eines Diens mit zwei konjugierten Doppelbindungen;
b) Zugabe eines Phosphinliganden und einer Katalysatorvorstufe, welche ausgewählt ist aus Palladiumdichlorid, Palladiumdibromid, Palladiumdiiodid, Palladium(II)-acetylacetonat, Palladium(II)-acetat, Bis-(di-benzylidene-aceton)-palladium, Bis(acetonitrile)-dichloro-palladium(II), Palladium-(cinnamyl)-dichlorid;
c) Zugabe eines Alkohols;
c') Zugabe einer Säure zum Reaktionsgemisch;
d) Zuführen von CO;
e) Erwärmen des Reaktionsgemisches, wobei das Dien zu einem Di- oder Tricarbonsäureester umgesetzt wird;
wobei der Phosphinligand eine Verbindung gemäß Formel (**I**) ist 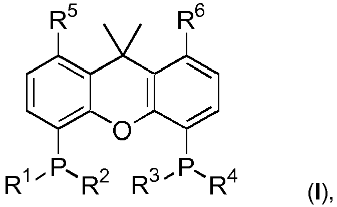 wobei
R¹, R², R³, R⁴ ausgewählt sind aus -(C₁-C₁₂)-Alkyl, -(C₆-C₂₀)-Aryl, -(C₃-C₁₂)-Cycloalkyl, -(C₃-C₁₂)-Heterocycloalkyl, -(C₃-C₂₀)-Heteroaryl;
R⁵, R⁶ ausgewählt sind aus -H, -(C₁-C₁₂)-Alkyl, -(C₆-C₂₀)-Aryl, -(C₃-C₁₂)-Cycloalkyl, -(C₃-C₁₂)-Heterocycloalkyl, -(C₃-C₂₀)-Heteroaryl;
und R¹, R², R³, R⁴, R⁵, R⁶, falls diese für -(C₁-C₁₂)-Alkyl, -(C₆-C₂₀)-Aryl, -(C₃-C₁₂)-Cycloalkyl, -(C₃-C₁₂)-Heterocycloalkyl oder -(C₃-C₂₀)-Heteroaryl stehen, jeweils unabhängig voneinander mit einem oder mehreren Substituenten ausgewählt aus -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl, -COO-(C₁-C₁₂)-Alkyl, -COO-(C₃-C₁₂)-Cycloalkyl, -CONH-(C₁-C₁₂)-Alkyl, - CONH-(C₃-C₁₂)-Cycloalkyl, -CO-(C₁-C₁₂)-Alkyl, -CO-(C₃-C₁₂)-Cycloalkyl, -N-[(C₁-C₁₂)-Alkyl]₂, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, - (C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, -COOH, -OH, -SO₃H, -NH₂, Halogen substituiert sein können.

2. Verfahren nach Anspruch 1,
wobei R¹, R², R³, R⁴ ausgewählt sind aus -(C₁-C₁₂)-Alkyl, -(C₆-C₂₀)-Aryl, -(C₃-C₂₀)-Heteroaryl.

3. Verfahren nach einem der Ansprüche 1 bis 2,
wobei R⁵, R⁶ ausgewählt sind aus -H, -(C₁-C₁₂)-Alkyl.

4. Verfahren nach einem der Ansprüche 1 bis 3,
wobei R¹, R², R³, R⁴ jeweils -(C₆-C₂₀)-Aryl sind und R⁵, R⁶ jeweils ausgewählt sind aus -H, -(C₁-C₁₂)-Alkyl.

5. Verfahren nach einem der Ansprüche 1 bis 4,
wobei das Dien 4 bis 30 Kohlenstoffatome umfasst.

6. Verfahren nach einem der Ansprüche 1 bis 5,
wobei das Dien ausgewählt ist aus Verbindungen der Formel (**II**) 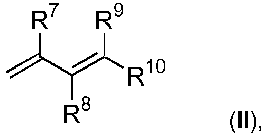 wobei R⁷, R⁸, R⁹, R¹⁰ unabhängig voneinander ausgewählt sein können aus -H, -(C₁-C₁₂)-Alkyl und -(C₆-C₂₀)-Aryl.

7. Verfahren nach einem der Ansprüche 1 bis 6,
wobei die Katalysatorvorstufe ausgewählt ist aus Palladiumdichlorid, Palladiumdibromid, Palladiumdiiodid, Palladium(II)-acetylacetonat, Palladium(II)-acetat, Bis-(di-benzylidene-aceton)-palladium.

8. Verfahren nach einem der Ansprüche 1 bis 7,
wobei die Katalysatorvorstufe ausgewählt ist aus Palladiumdichlorid, Palladiumdibromid, Palladiumdiiodid.

9. Verfahren nach einem der Ansprüche 1 bis 8,
wobei der Alkohol in Verfahrensschritt c) 1 bis 12 Kohlenstoffatome umfasst.

10. Verfahren nach einem der Ansprüche 1 bis 9,
wobei der Alkohol in Verfahrensschritt c) ausgewählt ist aus Methanol, Ethanol, 1-Propanol, iso-Propanol, n-Butanol, iso-Butanol, sec-Butanol, tert-Butanol, 1-Pentanol, 2-Pentanol, 3-Pentanol, 1-Hexanol, Cyclohexanol, 2-Ethylhexanol, iso-Nonanol, 2-Propylheptanol, Benzylalkohol, 1-Phenylethanol oder 2-Phenylethanol.

11. Verfahren nach einem der Ansprüche 1 bis 10,
wobei der Alkohol in Verfahrensschritt c) ausgewählt ist aus Methanol, Ethanol, iso-Propanol, n-Butanol oder 2-Phenylethanol.

## Claims

1. Method comprising the method steps of:
a) initially charging a diene having two conjugated double bonds;
b) adding a phosphine ligand and a catalyst precursor selected from palladium dichloride, palladium dibromide, palladium diiodide, palladium(II) acetylacetonate, palladium(II) acetate, bis(dibenzylideneacetone)palladium, bis(acetonitrile)dichloropalladium(II), palladium (cinnamyl) dichloride;
c) adding an alcohol;
c') adding an acid to the reaction mixture;
d) feeding in CO;
d) heating the reaction mixture, with conversion of the diene to a di- or tricarboxylic ester;
wherein the phosphine ligand is a compound according to formula (**I**) 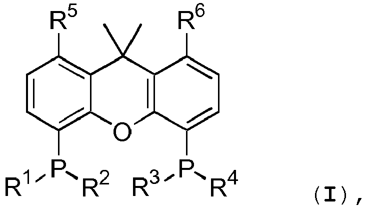 where
R¹, R², R³, R⁴ are selected from -(C₁-C₁₂)-alkyl, - (C₆-C₂₀)-aryl, -(C₃-C₁₂)-cycloalkyl, -(C₃-C₁₂)-heterocycloalkyl, -(C₃-C₂₀)-heteroaryl;
R⁵, R⁶ are selected from -H, - (C₁-C₁₂)-alkyl, -(C₆-C₂₀)-aryl, -(C₃-C₁₂)-cycloalkyl, -(C₃-C₁₂)-heterocycloalkyl, - (C₃-C₂₀)-heteroaryl;
and R¹, R², R³, R⁴, R⁵, R⁶, in the case that these are -(C₁-C₁₂)-alkyl, -(C₆-C₂₀) -aryl, -(C₃-C₁₂)-cycloalkyl, -(C₃-C₁₂)-heterocycloalkyl or -(C₃-C₂₀)-heteroaryl, may be each substituted independently of one another by one or more substituents selected from -(C₁-C₁₂)-alkyl, -(C₃-C₁₂)-cycloalkyl, -(C₃-C₁₂)-heterocycloalkyl, -O- (C₁-C₁₂) -alkyl, -O- (C₁-C₁₂)-alkyl-(C₆-C₂₀) -aryl, -O-(C₃-C₁₂)-cycloalkyl, -S-(C₁-C₁₂)-alkyl, -S- (C₃-C₁₂)-cycloalkyl, -COO- (C₁-C₁₂) - alkyl, -COO- (C₃-C₁₂)-cycloalkyl, -CONH- (C₁-C₁₂) - alkyl, -CONH-(C₃-C₁₂)-cycloalkyl, -CO- (C₁-C₁₂) -alkyl, -CO-(C₃-C₁₂)-cycloalkyl, -N-[(C₁-C₁₂)-alkyl]₂, -(C₆-C₂₀)-aryl, -(C₆-C₂₀) -aryl-(C₁-C₁₂)-alkyl, -(C₆-C₂₀)-aryl-O-(C₁-C₁₂) -alkyl, -(C₃-C₂₀)-heteroaryl, -(C₃-C₂₀)-heteroaryl-(C₁-C₁₂) -alkyl, -(C₃-C₂₀)-heteroaryl-O-(C₁-C₁₂)-alkyl, -COOH, -OH, -SO₃H, -NH₂, halogen.

2. Method according to Claim 1,
where R¹, R², R³, R⁴ are selected from - (C₁-C₁₂) -alkyl, - (C₆-C₂₀) -aryl, - (C₃-C₂₀)-heteroaryl.

3. Method according to either of Claims 1 to 2, where R⁵, R⁶ are selected from -H, -(C₁-C₁₂) -alkyl.

4. Method according to any of Claims 1 to 3, where R¹, R², R³, R⁴ are each -(C₆-C₂₀) -aryl and R⁵, R⁶ are each selected from -H, -(C₁-C₁₂) -alkyl.

5. Method according to any of Claims 1 to 4, wherein the diene comprises 4 to 30 carbon atoms.

6. Method according to any of Claims 1 to 5, wherein the diene is selected from compounds of the formula (**II**) 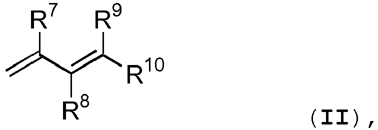 where R⁷, R⁸, R⁹, R¹⁰ may each independently be selected from -H, -(C₁-C₁₂)-alkyl and -(C₆-C₂₀)-aryl.

7. Method according to any of Claims 1 to 6,
wherein the catalyst precursor is selected from palladium dichloride, palladium dibromide, palladium diiodide, palladium(II) acetylacetonate, palladium(II) acetate, bis(dibenzylideneacetone)palladium.

8. Method according to any of Claims 1 to 7,
wherein the catalyst precursor is selected from palladium dichloride, palladium dibromide, palladium diiodide.

9. Method according to any of Claims 1 to 8,
wherein the alcohol in method step c) comprises 1 to 12 carbon atoms.

10. Method according to any of Claims 1 to 9,
wherein the alcohol in method step c) is selected from methanol, ethanol, 1-propanol, isopropanol, n-butanol, isobutanol, sec-butanol, tert-butanol, 1-pentanol, 2-pentanol, 3-pentanol, 1-hexanol, cyclohexanol, 2-ethylhexanol, isononanol, 2-propylheptanol, benzyl alcohol, 1-phenylethanol or 2-phenylethanol.

11. Method according to any of Claims 1 to 10,
wherein the alcohol in method step c) is selected from methanol, ethanol, isopropanol, n-butanol or 2-phenylethanol.

## Revendications

1. Procédé comprenant les étapes de procédé suivantes :
a) le chargement d'un diène contenant deux doubles liaisons conjuguées ;
b) l'ajout d'un ligand phosphine et d'un précurseur de catalyseur, qui est choisi parmi le dichlorure de palladium, le dibromure de palladium, le diiodure de palladium, l'acétylacétonate de palladium (II), l'acétate de palladium (II), le bis-(di-benzylidène-acétone)-palladium, le bis(acétonitrile)-dichloro-palladium (II), le dichlorure de palladium-(cinnamyle) ;
c) l'ajout d'un alcool ;
c') l'ajout d'un acide au mélange réactionnel ;
d) l'introduction de CO ;
e) le chauffage du mélange réactionnel, le diène étant transformé en un ester d'acide di- ou tricarboxylique ; le ligand phosphine étant un composé selon la formule (I) 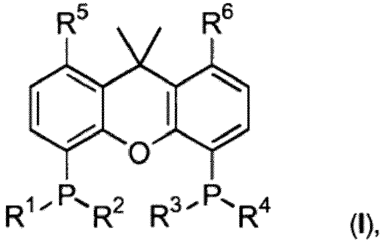 dans laquelle
R¹, R², R³, R⁴ sont choisis parmi alkyle en (C₁-C₁₂), aryle en (C₆-C₂₀), cycloalkyle en (C₃-C₁₂), hétérocycloalkyle en (C₃-C₁₂), hétéroaryle en (C₃-C₂₀);
R⁵, R⁶ sont choisis parmi H, alkyle en (C₁-C₁₂), aryle en (C₆-C₂₀), cycloalkyle en (C₃-C₁₂), hétérocycloalkyle en (C₃-C₁₂), hétéroaryle en (C₃-C₂₀);
et R¹, R², R³, R⁴, R⁵, R⁶, s'ils représentent alkyle en (C₁-C₁₂), aryle en (C₆-C₂₀), cycloalkyle en (C₃-C₁₂), hétérocycloalkyle en (C₃-C₁₂)ou hétéroaryle en (C₃-C₂₀), peuvent chacun indépendamment les uns des autres être substitués avec un ou plusieurs substituants choisis parmi alkyle en (C₁-C₁₂) , cycloalkyle en (C₃-C₁₂), hétérocycloalkyle en (C₃-C₁₂), -O-alkyle en (C₁-C₁₂), -O-alkyle en (C₁-C₁₂) -aryle en (C₆-C₂₀), -O-cycloalkyle en (C₃-C₁₂), -S-alkyle en (C₁-C₁₂), -S-cycloalkyle en (C₃-C₁₂), -COO-alkyle en (C₁-C₁₂), -COO-cycloalkyle en (C₃-C₁₂), - CONH-alkyle en (C₁-C₁₂), -CONH-cycloalkyle en (C₃-C₁₂), - CO-alkyle en (C₁-C₁₂), -CO-cycloalkyle en (C₃-C₁₂), -N-[alkyle en (C₁-C₁₂)]₂, aryle en (C₆-C₂₀), -aryle en (C₆-C₂₀)-alkyle en (C₁-C₁₂), aryle en (C₆-C₂₀) -O-alkyle en (C₁-C₁₂), hétéroaryle en (C₃-C₂₀), hétéroaryle en (C₃-C₂₀)-alkyle en (C₁-C₁₂), hétéroaryle en (C₃-C₂₀)-O-alkyle en (C₁-C₁₂), -COOH, -OH, -SO₃H, -NH₂, halogène.

2. Procédé selon la revendication 1, dans lequel R¹, R², R³, R⁴ sont choisis parmi alkyle en (C₁-C₁₂), aryle en (C₆-C₂₀), hétéroaryle en (C₃-C₂₀).

3. Procédé selon l'une quelconque des revendications 1 à 2, dans lequel R⁵, R⁶ sont choisis parmi H, alkyle en (C₁-C₁₂) .

4. Procédé selon l'une quelconque des revendications 1 à 3, dans lequel R¹, R², R³, R⁴ représentent chacun aryle en (C₆-C₂₀), et R⁵, R⁶ sont chacun choisis parmi H, alkyle en (C₁-C₁₂).

5. Procédé selon l'une quelconque des revendications 1 à 4, dans lequel le diène comprend 4 à 30 atomes de carbone.

6. Procédé selon l'une quelconque des revendications 1 à 5, dans lequel le diène est choisi parmi les composés de formule (II) 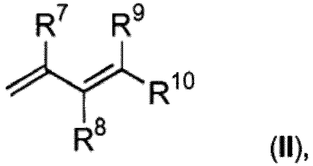 dans laquelle R⁷, R⁸, R⁹, R¹⁰ peuvent être choisis indépendamment les uns des autres parmi H, alkyle en (C₁-C₁₂) et aryle en (C₆-C₂₀).

7. Procédé selon l'une quelconque des revendications 1 à 6, dans lequel le précurseur de catalyseur est choisi parmi le dichlorure de palladium, le dibromure de palladium, le diiodure de palladium, l'acétylacétonate de palladium (II), l'acétate de palladium (II), le bis-(di-benzylidène-acétone)-palladium.

8. Procédé selon l'une quelconque des revendications 1 à 7, dans lequel le précurseur de catalyseur est choisi parmi le dichlorure de palladium, le dibromure de palladium, le diiodure de palladium.

9. Procédé selon l'une quelconque des revendications 1 à 8, dans lequel l'alcool à l'étape de procédé c) comprend 1 à 12 atomes de carbone.

10. Procédé selon l'une quelconque des revendications 1 à 9, dans lequel l'alcool à l'étape de procédé c) est choisi parmi le méthanol, l'éthanol, le 1-propanol, l'isopropanol, le n-butanol, l'iso-butanol, le sec-butanol, le tert-butanol, le 1-pentanol, le 2-pentanol, le 3-pentanol, le 1-hexanol, le cyclohexanol, le 2-éthylhexanol, l'iso-nonanol, le 2-propylheptanol, l'alcool benzylique, le 1-phényléthanol ou le 2-phényléthanol.

11. Procédé selon l'une quelconque des revendications 1 à 10, dans lequel l'alcool à l'étape de procédé c) est choisi parmi le méthanol, l'éthanol, l'isopropanol, le n-butanol ou le 2-phényléthanol.
